# EUROPÄISCHE PATENTSCHRIFT

(11) **EP 2 446 177 B1**
(45) Veröffentlichungstag und Bekanntmachung des Hinweises auf die Patenterteilung: **11.09.2013**
(21) Anmeldenummer: 10752267.4
(22) Anmeldetag: 23.06.2010
(51) Int. Cl.: F16K 15/14

(54) **MEMBRANVENTIL**
MEMBRANE VALVE
VANNE À MEMBRANE

(30) Priorität: 24.06.2009 DE 102009030186
(43) Veröffentlichungstag der Anmeldung: 02.05.2012
(73) Patentinhaber: Unither Therapeutik GmbH, 63820 Elsenfeld (DE)
(72) Erfinder: Kern, Joachim, 63820 Elsenfeld (DE)
(74) Vertreter: HOFFMANN EITLE
(86) Internationale Anmeldenummer: PCT/DE2010/000716
(87) Internationale Veröffentlichungsnummer: WO 2010/149145

(56) Entgegenhaltungen:
- EP-A1- 0 732 301
- DE-B- 1 163 107
- JP-A- 5 177 904
- US-A- 3 295 547
- US-B1- 7 100 637

## Beschreibung

Die Erfindung bezieht sich auf ein Membranventil insbesondere für Inhalationsgeräte, bestehend aus einer flächigen Membrane aus dauerhaft flexiblem Material und einem Rahmen, der eine Luftöffnung aufweist, die etwas kleiner als die Membrane ist und wenigstens einem Steg, der den Rahmen mit einer Halterung verbindet, die etwa in der Mitte der Luftöffnung angeordnet ist, und einem Stift, dessen Schaft durch eine Befestigungsöffnung etwa in der Mitte der Membrane verläuft und sich in einer Halteöffnung in der Halterung befindet, wobei die Membrane im Ruhezustand auf dem Rahmen aufliegt.

In den verschiedensten Systemen zur Führung der Atemluft von Menschen und anderen Lebewesen sind Ventile erforderlich. Eine häufige Aufgabe ist es, dem Lebewesen ein ungehindertes Ausatmen zu ermöglichen, wozu sich eine Membrane durch den Druck des vom Lebewesen ausgestoßenen Atems von einem Rahmen abhebt, auf dem sie im Ruhezustand aufliegt. Dann kann durch eine Luftöffnung im Rahmen die Atemluft austreten. Wenn das Lebewesen wieder einatmet, kehrt sich die Richtung des Luftstromes um, sodass der Druck auf die Membrane entfällt und sie sich durch die Elastizität ihres Materials wieder in den Ruhestand zurückbewegt, in dem sie durch Aufliegen auf dem Rahmen den Zustrom von Gasen und Flüssigkeiten blockiert. Stattdessen wird Atemluft aus einer anderen Quelle zugeführt, wie z. B. aus einem Drucktank oder aus der Vernebelungskammer eines Inhalationsgerätes zur Beaufschlagung der Atemluft mit vernebelten Wirkstoffen. Dabei ist es eine wesentliche Funktion des Ventils, dass es nicht nur den Zustrom von Gasen blockiert, sondern auch das Eindringen von Flüssigkeiten verhindert.

Auf aktuellem Stand der Technik zeigt die GS DE 20 2004 021 350 ein Auslassventil, mit dem menschliche Atemluft eines Tauchers in das umgebende Wasser ausgestoßen wird. Dazu liegt eine Membrane auf dem Rahmen um eine Luftöffnung herum auf und wird in der Mitte des Rahmens in einer Halterung gehalten, die von Stegen getragen wird. In dieser Halterung ist die Membrane mit einem Stift befestigt.

Beim Ausstoßen der Atemluft wird die elastische Membrane vom Rahmen abgehoben, sodass Luft austreten kann. Wenn der Druck der herausdrängenden Atemluft entfällt, bewegt die Elastizität der Membrane diese wieder an den Rahmen zurück.

Ein wesentlicher Nachteil dieser Anordnung ist die begrenzte Dichtwirkung der Membrane gegen das Eindringen von Wasser. Um einen höheren Anpressdruck zu erzeugen, ist der Rand der scheibenförmigen Membrane am Rand umlaufend etwas abgeknickt, sodass die Membrane nicht mehr flächig auf dem Rahmen aufliegt, sondern nur noch mit der Kante. Dadurch wird die Auflagefläche dramatisch verkleinert, sodass in dem verbleibenden, ringförmigen Bereich der Anpressdruck der Kante der Membrane relativ hoch ist. Der wesentliche, sich daraus ergebende Nachteil ist jedoch, dass die Atemluft einen erhöhten Druck aufbringen muss, um die Membrane zu bewegen, da sie durch ihre Profilierung relativ steif geworden ist.

Als eine andere Variante sind scheibenförmige Membranen bekannt, die die Form eines Ringes haben, der aus plattenförmigem, elastischem Material ausgeschnitten ist. Dieser Ring setzt der Atemluft einen vergleichsweise geringeren Widerstand entgegen.

Sein wesentlicher Nachteil ist jedoch, dass er auf seinem festen Gegenstück - wie z. B. einem Rahmen - nur aufliegt, wodurch sich kapillarförmige Schlitze bilden. Wenn zu diesen Schlitzen Flüssigkeiten gelangen, so werden sie durch die Kapillarwirkung gefördert, so dass sich über eine längere Zeit hinweg ein kleiner, aber steter Flüssigkeitsstrom ausbildet.

Ferner sind aus der gattungsbildenden EP-A-0 732 301, der US-A-7,100,637, der DE-A-11 63 107, der US-A-3,295,547 oder der JP-A-05-177904 Stifte zum Befestigen einer Membran bekannt, die einen verbreiterten Kopf aufweisen.

Auf diesem Hintergrund hat sich die Erfindung die Aufgabe gestellt, ein Membranventil zu entwickeln, dessen Membrane im Ruhezustand mit einer relativ hohen Kraft an den feststehenden Rahmen gedrückt wird, das aber der hindurchtretenden Luft nur einen sehr kleinen Widerstand entgegen setzt.

Als Lösung präsentiert die Erfindung die Merkmale des Anspruchs 1. Ausführungsformen der Erfindung sind in den Unteransprüchen genannt.

Das wesentliche Merkmal der Erfindung ist also der in der Nähe des Kopfes vergrößerte Schaft des Stiftes zur Befestigung des Membranventils. Die dadurch erreichte, vorteilhafte Wirkung lässt sich am besten im Querschnitt der Membrane erläutern. In der äußeren Schicht der Membrane, die vom Rahmen weiter entfernt ist, wird die Membrane durch den vergrößerten Durchmesser des Schaftes auseinandergedrückt. Dadurch entstehen von der Mitte der Außenfläche der Membrane ausgehende radial wirkende Kräfte, die das dauerhaft flexible Material der Membrane nach außen drücken. Der innere, zum Rahmen weisende Teil der Membrane wird nicht belastet, da der Durchmesser des Schaftes in diesem Bereich nur so groß ist wie die Befestigungsöffnung in der Membrane. Dadurch wird die Membrane gewölbt, es wird ihr also eine Kalottenform (mit einem relativ großen Durchmesser) verliehen. Dadurch liegt die Membrane nur mit ihrer äußeren Kante auf dem Rahmen auf, aber mit einem dadurch erhöhten Anpressdruck und erzielt so eine Abdichtung gegen Flüssigkeit, die von außen eindringt.

Damit die Schaftvergrößerung in erfindungsgemäßer Weise wirksam wird, muss sie sich innerhalb der Befestigungsöffnung der Membran befinden - z.B. bei einer zylindrischen Befestigungsöffnung im Bereich der vom Rahmen abgewandten Seite. Dazu muss der Schaft des Stiftes in die passende Einstecktiefe gebracht werden.

Als eine Ausführungsvariante wird dafür ein Anschlag benannt, der das Erreichen der korrekten Einstecktiefe gewährleistet. Er ist innerhalb der Halteöffnung für den Schaft angeordnet und lässt den Schaft nur soweit in die Halteöffnung eindringen, dass der Abstand zwischen der zur Membran weisenden Fläche des Kopfes und der zur Membran weisenden Fläche der Halterung wenigstens so groß wie die Materialstärke der Membran ist.

Eine sinnvolle Form dieses Anschlages wird dadurch erreicht, dass die Halteöffnung für den Schaft des Stiftes als ein Sackloch ausgebildet wird, an dessen Boden der Stift anschlägt. Dabei ist es vorteilhaft, wenn die "Spitze" des Schaftes komplementär zum "Boden" des Sackloches geformt ist.

Im Vergleich zum bekannten Stand der Technik ist der entscheidende Vorteil dieser Anordnung, dass das Material der Membran seine ursprüngliche Flexibilität beibehält. Es wird nicht durch Profilierungen oder Verformungen versteift, sodass es der ausgeatmeten Luft fast den gleichen - geringen - Widerstand entgegensetzt, der bei der Befestigung der Membranscheibe mittels eines Stiftes mit zylindrischem Schaft ohne eine erfindungsgemäße Aufweitung in der Nähe des Kopfes möglich ist.

Ein wesentlicher Vorteil der Erfindung ist, dass bereits bekannte Membranen ohne jede Änderung der - meist aufwändigen - Form weiterhin hergestellt und verwendet werden können. Es ist sogar möglich, die Membrane aus plattenförmigem Material auszustanzen.

Dabei kann die Fläche der Membran eine beliebige Form einnehmen. Besonders vorteilhaft ist jedoch eine Kreisform, da sie im Verhältnis zur Fläche der Luftöffnung und damit dem maximal hindurch bewegbaren Atemluftvolumen die geringste Länge des Umfanges aufweist und damit die Vorspannung, die durch die Erweiterung des Schaftes vom Stift in der Nähe des Kopfes erreicht wird, auf die geringste mögliche Länge aufteilt, also den Flächendruck im Auflagenbereich maximiert und damit die beste Dichtungswirkung erzielt.

Damit die Verteilung dieser Vorspannungskraft etwa aus der Mitte der Membrane bis zu ihrem Rand möglichst gleichmäßig erfolgt, bevorzugt die Erfindung, dass beide Flächen der Membrane eben sind, weil in diesem Fall die Vorspannkraft in alle Richtungen der Membrane auch eine gleichmäßige Ausbildung der Kalottenform der Membrane bewirkt. Diese Eigenschaft wird z. B. dadurch erreicht, dass die Membrane - wie erwähnt - aus einem plattenförmigen Material ausgestanzt wird.

Die wünschenswerte, gleichmäßige Verteilung des Andruckes der Membrankante auf dem Rahmen wird in einer Ausführungsvariante dadurch unterstützt, dass die Befestigungsöffnung in der Membrane zylindrisch geformt und senkrecht zur rahmenseitigen Fläche der Membrane ausgerichtet ist. Dann ist es zu bevorzugen, dass der an den Kopf anschließende Teil des Schaftes als Kegelstumpf geformt ist, der in einen zylindrisch geformten Abschnitt des Schafts übergeht. Dieser letztere, zylindrische Abschnitt entspricht in seinem Durchmesser dem Durchmesser der Befestigungsöffnung und drückt nicht in radialer Richtung auf die Membrane.

Eine solche, radial gerichtete und nach allen Seiten gleichmäßig verteilte Kraft wird nur durch den Kegelstumpf in der Nähe des Kopfes ausgeübt. Da der Kegelstumpf direkt am Kopf seinen größten Durchmesser aufweist, ist dort auch die nach außen gerichtete Kraft am größten und nimmt mit zunehmender Entfernung vom Kopf des Schaftes ab. Wenn ein solcher Stift in die Befestigungsöffnung einer Membrane eingedrückt wird, die noch nicht auf dem Rahmen aufliegt, ergibt sich eine leicht kalottenförmige Verformung der Membrane. Dieser Effekt sorgt dafür, dass nur die Außenkante der Membrane ringförmig auf den Rahmen aufgedrückt wird, wenn die Membrane mit dem Stift in der Halteöffnung der Halterung inmitten der Luftöffnung befestigt wird.

Wenn die Membrane aus einem plattenförmigen Rohmaterial ausgestanzt wird, verläuft ihre Halteöffnung durch das Material hindurch. Diese Öffnung muss dann mit dem Kopf und dem Schaft des Stiftes wieder abgedichtet werden.

Wenn die Halteöffnung eine durchgehende Bohrung ist, muss deren Abdichtung gesichert sein. Dazu schlägt die Erfindung als eine Ausführungsvariante vor, dass an die Membrane um die Befestigungsöffnung herum ein Kragen angeformt wird, der in die Halteöffnung in der Halterung hineinragt. Dadurch bildet sich zwischen dem Schaft des Stiftes und der Halteöffnung der Kragen als zusätzliches Dichtelement aus. Wenn der Kragen durch ein geringes Übermaß des Schaftes noch zusätzlich an die Halteöffnung der Halterung gepresst wird, wird die Dichtwirkung verbessert.

In einer weiteren Variante wird der Kragen durch eine Kalotte, einen Kegelstumpf oder einen anderen Verschluss geschlossen. Damit ist eine perfekte Dichtwirkung der Membrane in ihrer Mitte garantiert. In diesem Fall sollte das freie Ende des Schaftes komplementär zum Kragen der Membrane geformt sein und darin eingepresst oder eingeklebt werden. Der Kragen wird dann seinerseits in die Halteöffnung der Halterung eingepresst oder eingeklebt. Durch diese beiden Press- oder Klebverbindungen wird die Membrane mit der Halterung sicher verbunden.

In der bereits erwähnten Ausführungsform der Membrane als Scheibe, die aus plattenförmigem Material ausgestanzt worden ist, kann das freie Ende des Schaftes in die Halteöffnung der Halterung eingepresst, eingeklebt, eingenietet, eingeschmolzen oder eingeschraubt werden. Dann übernimmt die Verbindung des Schaftes mit der Halteöffnung die mechanische Sicherung der Membrane. Für die einwandfreie Abdichtung der Befestigungsöffnung muss dann die Ausbildung des Kopfes und dessen Auflage auf der nach außen weisenden Fläche der Membrane sorgen.

Das erfindungsgemäße Membranventil ist eine wesentliche Verbesserung für Inhalationsgeräte, die eine Verneblungseinrichtung für flüssige Wirkstoffe und eine Führungseinrichtungen für die Atemluft von Lebewesen enthalten. Wenn die Führungseinrichtung für die Atemluft wenigstens ein erfindungsgemäßes Membranventil enthält, kann damit das unerwünschte Eindringen von Feuchtigkeit - z.B. aus der Atemluft in die Aerosolkammer - verhindert werden.

Andere interessante Anwendungen sind Beatmungsgeräte, Lungendiagnosegeräte, Atemmasken, Sauerstoffgeräte oder Tauchgeräte.

Im Folgenden sollen weitere Einzelheiten und Merkmale der Erfindung anhand eines Beispiels näher erläutert werden. Dieses soll die Erfindung jedoch nicht einschränken, sondern nur erläutern. Es zeigt in schematischer Darstellung:
- Figur 1: Schnitt durch ein Membranventil im Ruhezustand
- Figur 2: Schnitt durch Membranventil wie oben vor dem Einsetzen in den Rahmen
- Figur 3: Querschnitt durch eine Membrane vor der Montage
- Figur 4: Querschnitt durch ein Membranventil im geöffneten Zustand

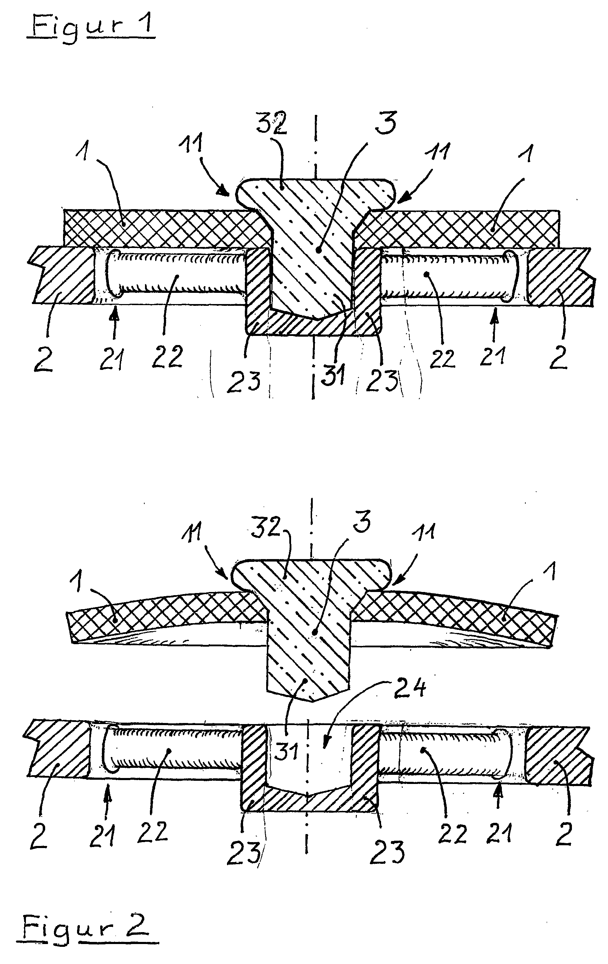

In Figur 1 ist der Schnitt durch ein erfindungsgemäßes Membranventil gezeichnet. Durch eine strichpunktierte Linie in der Mitte ist dargestellt, dass die gezeigte Ausführungsform rotationssymmetrisch ist. Ganz außen sind die beiden Schnittflächen durch den Rahmen (2) zu erkennen. Der Rahmen (2) umschließt die Luftöffnung (21), die in Figur 1 zweimal zu sehen ist, da sie in der Mitte durch die Halterung (23) unterbrochen wird. Die Halterung (23) wird - ähnlich wie bei einem Speichenrad - durch die Stege (22) mit dem Rahmen (2) verbunden, der die Luftöffnung (21) kreisförmig umgibt.

In Figur 1 ist die Schnittfläche durch die Membrane (1) kreuzschraffiert dargestellt. Die Membrane (1) liegt mit ihrem äußeren Rand auf dem Rahmen (2) auf. In Figur 2 wird dargestellt, wie sie in diesem Bereich durch ausströmende Atemluft vom Rahmen (2) abgehoben werden kann und dadurch einen Weg für das durchströmende Gas freigibt.

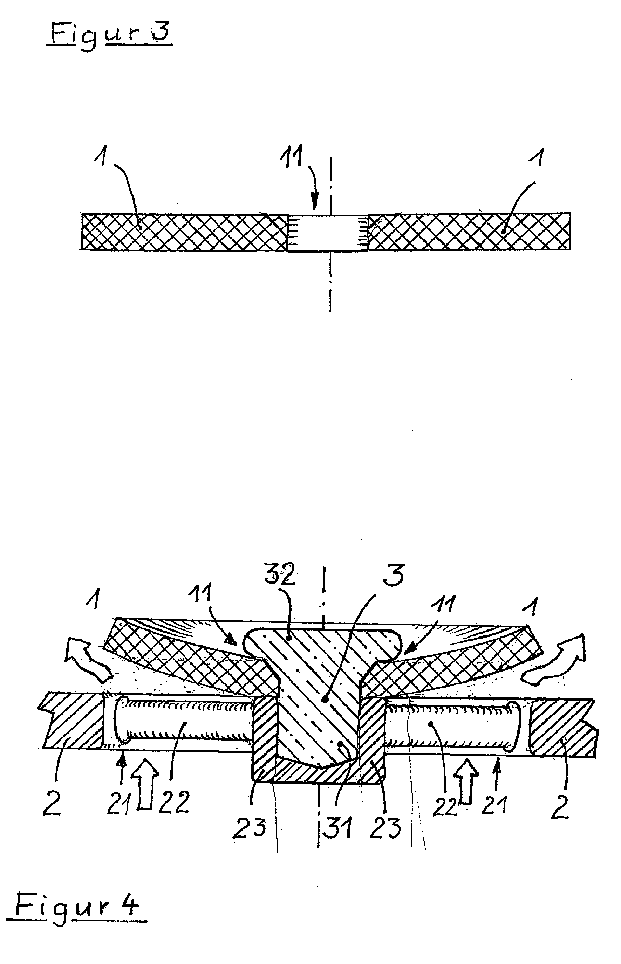

In Figur 1 ist in der Mitte der Membrane (1) durch Pfeile die Befestigungsöffnung (11) gekennzeichnet. In der hier dargestellten Ausführungsform ist in die Membrane eine zylindrische Befestigungsöffnung (11) eingebracht - wie in Figur 3 gezeigt wird.

In Figur 1 wird die Befestigungsöffnung (11) jedoch in dem Zustand dargestellt, in dem bereits ein der Stift (3) eingepresst. In Figur 1 ist sehr gut zu erkennen, dass er im unteren Bereich des Schaftes (31) zylindrisch verläuft. In diesem Bereich ist der Durchmesser des Schaftes (31) ebenso groß wie der Durchmesser der Befestigungsöffnung (11). Nur im oberen Bereich des Schaftes (31) in unmittelbarer Nähe des Kopfes (32) ist der Durchmesser des Schaftes stetig vergrößert. Dadurch wird auch die Befestigungsöffnung (11) der Membrane (1) auf einem Teil auseinander gedrückt.

Der Stift (3) übt also im Bereich des Querschnittes der Membrane (1) nahe der Außenfläche, die vom Rahmen weg weist, einen erhöhten Druck aus, der sich über den äußeren Bereich der Membrane (1) bis zu deren Rand hin fortpflanzt und für einen erhöhten Anpressdruck des Randes der Membrane (1) auf dem Rahmen (2) sorgt. Dadurch wird sichergestellt, dass in diesem Bereich eine rundum wirkende Dichtung aufgebaut wird, die auch evtl. eindringende Feuchtigkeit zurückhält.

In Figur 1 wird eine Ausführungsform der Halterung (23) mit einem Anschlag gezeigt. In dieser Variante ist die Halteöffnung (24) der Halterung (23) als Sackloch ausgebildet. Der Boden des Sackloches dient als Anschlag für den Stift. Als eine weitere Variante ist dessen Spitze komplementär zum Boden des Sackloches geformt.

In Figur 2 ist zur besseren Erläuterung der vorteilhaften Wirkung des verbreiterten Teiles vom Schaft (31) eine Membrane (1) mit bereits eingepresstem Stift (3) vor dem Einsetzen in den Rahmen (2) gezeichnet. Sehr gut zu erkennen ist, dass der verbreiterte Teil des Schaftes (31) auf den oberen Teil des Querschnittes von der Membrane (1) Druckkräfte ausübt, die bewirken, dass die Membrane (1) kalottenförmig gewölbt wird. Dadurch liegt die so entstandene Kalotte beim Einsetzen in den Rahmen (2) nur mit ihrer Kante auf dem Rahmen (2) auf.

In Figur 3 ist der Vollständigkeit halber zur Erläuterung der vorteilhaften Wirkung des Hauptmerkmales der Erfindung - nämlich der kegelförmigen Verbreiterung des Schaftes (31) vom Stift (3) - noch einmal die Membrane (1) gezeichnet, jedoch vor ihrer Montage. Deutlich wird erkennbar, dass die Befestigungsöffnung (11) in der Membrane (1) durchgehend zylindrisch geformt ist, wodurch insbesondere am Eingang der Befestigungsöffnung (11) eine scharfe Kante entsteht. Auf diese scharfe Kante drückt die kegelige Verbreiterung des Schaftes (31) vom Stift (3) beim Hereinpressen in die Befestigungsöffnung (11) und in den Kragen. Das dabei verdrängte Volumen der dauerhaft flexiblen Membrane (1) erzeugt die Kräfte im oberen Bereich des Querschnittes, die für einen erhöhten Anpressdruck des Randbereiches der Membrane (1) sorgen.

In Figur 4 ist das in Figur 1 gezeichnete Beispiel wiederholt, jedoch im Zustand von durchströmender Luft, die jeweils durch Doppelpfeile gekennzeichnet ist. Gut zu erkennen ist, wie die Luft durch die Luftöffnungen (21) des Rahmens (2) hindurchtritt, dabei an den Stegen (22) vorbeistreicht und auf die flexible Membrane (1) drückt, die dadurch angehoben wird, sodass sich zwischen dem außen umlaufenden Rahmen (2) und der Kante der Membrane (1) ein Schlitz öffnet, durch den die Luft entweicht.

In Figur 4 ist sehr gut zu erkennen, dass sich in diesem Betriebszustand die flexible Membrane (1) wiederum etwa kalottenförmig wölbt, jedoch nach oben hin. Es wird deutlich, dass die durch den Luftdruck auf die Membrane (1) ausgeübten Kräfte in diesem Betriebszustand größer sind, als die durch das Verdrängen des Materialvolumens an der oberen Kante der Membrane (1) in radialer Richtung ausgeübten Kräfte.

### Bezugszeichenliste

- 1: Membrane, flächig, dauerhaft flexibel
- 11: Befestigungsöffnung in Membrane 1
- 2: Rahmen, berührt Membrane 1
- 21: Luftöffnung, im Rahmen 2
- 22: Steg, vom Rahmen 2 zur Halterung 23 in der Mitte der Luftöffnung 21
- 23: Halterung, getragen von wenigstens einem Steg 22
- 24: Halteöffnung in Halterung 23
- 3: Stift
- 31: Schaft des Stiftes 3
- 32: Kopf des Stiftes 3

## Patentansprüche

1. Membranventil insbesondere für Inhalationsgeräte, bestehend aus
- einer flächigen Membrane (1) aus dauerhaft flexiblem Material und
- einem Rahmen (2), der eine Luftöffnung (21) aufweist, die etwas kleiner als die Membrane (1) ist und
- wenigstens einem Steg (22), der den Rahmen (2) mit
- einer Halterung (23) verbindet, die etwa in der Mitte der Luftöffnung (21) angeordnet ist, und
- einem Stift (3), dessen Schaft (31)
- durch eine Befestigungsöffnung (11) etwa in der Mitte der Membrane (1) verläuft und
- sich in einer Halteöffnung (24) in der Halterung (23) befindet,
wobei
- der Stift (3) zu einem Kopf (32) verbreitert ist,
- dessen Abstand zur Membrane (1) klein im Vergleich zur Stärke der Membrane (1) ist oder
- der auf der Membrane (1) aufliegt und wobei
- die Membrane (1) im Ruhezustand auf dem Rahmen (2) aufliegt
**dadurch gekennzeichnet, dass**
- der Durchmesser des Schaftes (31)
- in der Nähe des Kopfes (32) grösser als der Durchmesser der Befestigungsöffnung (11) ist und
- am freien Ende des Schaftes (31) etwa dem Durchmesser der Befestigungsöffnung (11) gleicht, so dass in einem äußeren Teil der Membrane, die vom Rahmen weiter entfernt ist, die Membrane durch den vergrößerten Durchmesser des Schafts auseinander gedrückt wird, während ein innerer zum Rahmen weisender Teil der Membrane nicht belastet wird, wodurch die Membrane gewölbt wird.

2. Membranventil nach dem vorhergehenden Anspruch 1, **dadurch gekennzeichnet, dass** innerhalb der Halteöffnung (24) ein Anschlag für den Schaft (31) angeordnet ist, der den Schaft (31) nur soweit in die Halteöffnung (24) eindringen lässt, dass der Abstand zwischen der zur Membran (1) weisenden Fläche des Kopfes (31) und der zur Membran (1) weisenden Fläche der Halterung (23) wenigstens so groß wie die Materialstärke der Membran (1) ist.

3. Membranventil nach einem der vorhergehenden Ansprüche, **dadurch gekennzeichnet, dass** die Fläche der Membran (1) kreisförmig ist.

4. Membranventil nach einem der vorhergehenden Ansprüche, **dadurch gekennzeichnet, dass** beide Flächen der Membran (1) eben sind.

5. Membranventil nach einem der vorhergehenden Ansprüche, **dadurch gekennzeichnet, dass** die Befestigungsöffnung (11) zylindrisch geformt und senkrecht zur rahmenseitigen Fläche der Membran (1) ausgerichtet ist und der an den Kopf (32) anschließende Teil des Schaftes (31) als Kegelstumpf geformt ist, der in einen zylindrisch geformten Abschnitt des Schaftes (31) übergeht.

6. Membranventil nach einem der vorhergehenden Ansprüche, **dadurch gekennzeichnet, dass** an die Membrane (1) um die Befestigungsöffnung (11) herum ein Kragen angeformt ist, der in die Halteöffnung (24) in der Halterung (23) hineinragt.

7. Membranventil nach Anspruch 6, **dadurch gekennzeichnet, dass** an den Kragen
- eine Kalotte oder
- ein Kegelstumpf oder
ein anderer Verschluss angeformt ist.

8. Membranventil nach einem der vorhergehenden Ansprüche, **dadurch gekennzeichnet, dass** das freie Ende des Schaftes (31)
- komplementär zum Kragen der Membran (1) geformt ist und
- darin eingepresst oder eingeklebt ist und
- der Kragen in die Halteöffnung (24) der Halterung (23) eingepresst oder eingeklebt ist.

9. Membranventil nach einem der vorhergehenden Ansprüche, **dadurch gekennzeichnet, dass** das freie Ende des Schaftes (31) in die Halteöffnung (24)
- eingepresst und/oder
- eingeklebt und/oder
- eingenietet und/oder
- eingeschmolzen und/oder
- eingeschraubt ist.

10. Inhalationsgerät, enthaltend
- eine Verneblungseinrichtung für flüssige Wirkstoffe und
- Führungseinrichtungen für die Atemluft von Lebewesen,
**dadurch gekennzeichnet, dass**
die Führungseinrichtung für die Atemluft wenigstens ein Membranventil nach einem der vorherigen Ansprüche enthält.

11. Anwendung eines Membranventils nach einem der vorhergehenden Ansprüche in
- einem Inhalationsgerät oder
- einem Beatmungsgerät oder
- einem Lungendiagnosegerät oder
- einer Atemmaske oder
- einem Sauerstoffgerät oder
- einem Tauchgerät.

## Claims

1. Diaphragm valve in particular for inhalation devices, consisting of
- a two-dimensional diaphragm (1) made of permanently flexible material and
- a frame (2) which has an air opening (21) which is slightly smaller than the diaphragm (1) and
- at least one web (22) which connects the frame (2) to
- a support (23) which is disposed approximately in the middle of the air opening (21), and
- a pin (3) of which the shaft (31)
- runs through a fastening opening (11) approximately in the middle of the diaphragm (1) and
- is located in a holding opening (24) in the support (23),
wherein
- the pin (3) widens into a head (32)
- of which the distance from the diaphragm (1) is small compared with the thickness of the diaphragm (1) or
- which rests on the diaphragm (1) and wherein
- the diaphragm (1) in the resting state rests on the frame (2),
**characterised in that**
- the diameter of the shaft (31)
- in the vicinity of the head (32) is larger than the diameter of the fastening opening (11) and
- at the free end of the shaft (31) is approximately equal to the diameter of the fastening opening (11), so that in an outer portion of the diaphragm which is further away from the frame, the diaphragm is pushed apart by the increased diameter of the shaft, while an inner portion of the diaphragm facing the frame is not stressed, as a result of which the diaphragm is cambered.

2. Diaphragm valve according to the preceding claim 1, **characterised in that** within the holding opening (24) is disposed a stop for the shaft (31), which lets the shaft (31) enter the holding opening (24) only so far that the distance between the surface of the head (31) facing the diaphragm (1) and the surface of the support (23) facing the diaphragm (1) is at least as great as the material thickness of the diaphragm (1).

3. Diaphragm valve according to any of the preceding claims, **characterised in that** the surface of the diaphragm (1) is circular.

4. Diaphragm valve according to any of the preceding claims, **characterised in that** both surfaces of the diaphragm (1) are flat.

5. Diaphragm valve according to any of the preceding claims, **characterised in that** the fastening opening (11) is cylindrically shaped and oriented perpendicularly to the surface of the diaphragm (1) on the frame side, and the portion of the shaft (31) adjoining the head (32) is shaped as a truncated cone which merges with a cylindrically shaped section of the shaft (31).

6. Diaphragm valve according to any of the preceding claims, **characterised in that** on the diaphragm (1) round the fastening opening (11) is integrally formed a collar which extends into the holding opening (24) in the support (23).

7. Diaphragm valve according to claim 6, **characterised in that** on the collar is integrally formed
- a spherical cap or
- a truncated cone or
some other fastening.

8. Diaphragm valve according to any of the preceding claims, **characterised in that** the free end of the shaft (31)
- is shaped complementarily to the collar of the diaphragm (1) and
- pressed or adhered therein and
- the collar is pressed or adhered into the holding opening (24) of the support (23).

9. Diaphragm valve according to any of the preceding claims, **characterised in that** the free end of the shaft (31) is
- pressed and/or
- adhered and/or
- riveted and/or
- melted and/or
- screwed
into the holding opening (24).

10. Inhalation device, containing
- a nebuliser device for liquid active ingredients and
- guide means for the respiratory air of living creatures, **characterised in that** the guide means for the respiratory air includes at least one diaphragm valve according to any of the preceding claims.

11. Use of a diaphragm valve according to any of the preceding claims in
- an inhalation device or
- a respirator or
- a lung diagnosis device or
- a breathing mask or
- an oxygen device or
- diving equipment.

## Revendications

1. Valve à membrane, en particulier pour inhalateurs, composée
- d'une membrane (1) plate en matériau durablement flexible, et
- d'un cadre (2) présentant une ouverture de ventilation (21) légèrement plus petite que la membrane (1), et
- d'au moins une entretoise (22) reliant le cadre (2) à
- un support (23) disposé sensiblement au centre de l'ouverture de ventilation (21), et
- d'une goupille (3), dont la tige (31)
- s'étend sensiblement au centre de la membrane (1) au travers d'une ouverture de fixation (11), et
- se trouve dans une ouverture de maintien (24) du support (23),
- la goupille (3) s'élargissant sous forme de tête (32),
- dont l'espacement à la membrane (1) est réduit en comparaison de l'épaisseur de la membrane (1), ou
- qui s'appuie sur la membrane (1) et
- la membrane (1) s'appuyant sur le cadre (2) en état de repos,
**caractérisée en ce que**
- le diamètre de la tige (31)
- est supérieur à proximité de la tête (32) au diamètre de l'ouverture de fixation (11), et
- correspond sensiblement au diamètre de l'ouverture de fixation (11) à l'extrémité libre de la tige (31), si bien que, dans une partie extérieure de la membrane, plus distante du cadre, la membrane est contrainte par le diamètre agrandi de la tige, tandis qu'une partie intérieure de la membrane opposée au cadre n'est pas contrainte, ce qui cintre la membrane.

2. Valve à membrane selon la revendication 1, **caractérisée en ce qu'**une butée pour la tige (31) est disposée à l'intérieur de l'ouverture de maintien (24), laquelle ne laisse pénétrer la tige (31) dans l'ouverture de maintien (24) que jusqu'à ce que l'espacement entre la face de la tête (31) opposée à la membrane (1) et la face du support (23) opposée à la membrane (1) soit au moins égale à l'épaisseur de matière de la membrane (1).

3. Valve à membrane selon l'une des revendications précédentes, **caractérisée en ce que** la surface de membrane (1) est circulaire.

4. Valve à membrane selon l'une des revendications précédentes, **caractérisée en ce que** les deux faces de la membrane (1) sont planes.

5. Valve à membrane selon l'une des revendications précédentes, **caractérisée en ce que** l'ouverture de fixation (11) est de forme cylindrique et est ménagée perpendiculairement à la face de membrane (1) côté cadre, et **en ce que** la partie de tige (31) adjacente à la tête (32) est de forme tronconique contiguë à une partie de forme cylindrique de la tige (31).

6. Valve à membrane selon l'une des revendications précédentes, **caractérisée en ce qu'**un col est formé sur la membrane (1) tout autour de l'ouverture de fixation (11), lequel fait saillie dans l'ouverture de maintien (24) du support (23).

7. Valve à membrane selon la revendication 6, **caractérisée en ce que**
- une calotte ou
- un cône tronqué ou
une autre fermeture sont formés sur le col.

8. Valve à membrane selon l'une des revendications précédentes, **caractérisée en ce que** l'extrémité libre de la tige (31)
- présente une forme complémentaire au col de la membrane (1), et
- est chassée ou collée dans celle-ci, et
- le col est chassé ou collé dans l'ouverture de maintien (24) du support (23).

9. Valve à membrane selon l'une des revendications précédentes, **caractérisée en ce que** l'extrémité libre de la tige (31) est
- chassée et/ou
- collée et/ou
- rivetée et/ou
- fondue et/ou.
- vissée
dans l'ouverture de maintien (24).

10. Inhalateur, comprenant
- un dispositif de nébulisation pour des substances actives liquides, et
- des dispositifs de guidage pour l'air inhalé par des êtres vivants,
**caractérisé en ce que**
le dispositif de guidage pour l'air inhalé comporte au moins une valve à membrane selon l'une des revendications précédentes.

11. Utilisation d'une valve à membrane selon l'une des revendications précédentes dans
- un inhalateur, ou
- un appareil respiratoire, ou
- un appareil de diagnostic pulmonaire, ou
- un masque respiratoire, ou
- un appareil à oxygène, ou
- un appareil de plongée.
